# EUROPÄISCHE PATENTSCHRIFT

(11) **EP 0 903 345 B1**
(45) Veröffentlichungstag und Bekanntmachung des Hinweises auf die Patenterteilung: **04.10.2000**
(21) Anmeldenummer: 98114373.8
(22) Anmeldetag: 31.07.1998
(51) Int. Cl.: C07D 261/18, A61K 31/42

(54) **Kristallform von 5-Methylisoxazol-4-carbonsäure-(4-trifluormethyl)-anilid**
Crystal form of N-(4-trifluoromethylphenyl)-5-methylisoxazole-4-carboxamid
Forme cristalline de N-(4-trifluorométhylphényl)-5-méthylisoxazole-4-carboxamid

(30) Priorität: 08.08.1997 DE 19734438; 17.12.1997 DE 19756093
(43) Veröffentlichungstag der Anmeldung: 24.03.1999
(60) Teilanmeldung: 99117098.6
(73) Patentinhaber: Aventis Pharma Deutschland GmbH, 65929 Frankfurt am Main (DE)
(72) Erfinder: Faasch, Holger, Dr., 65239 Hochheim (DE); Hedtmann, Udo, Dr., 60433 Frankfurt (DE); Westenfelder, Uwe, Dr., 65931 Frankfurt (DE); Paulus, Erich, Dr., 65817 Eppstein (DE)

(56) Entgegenhaltungen:
- EP-A- 0 013 376
- EP-A- 0 617 959
- WO-A-91/17748
- DE-A- 2 525 959
- DE-A- 4 127 737

## Beschreibung

Die Erfindung betrifft eine neue gut lösliche Kristallmodifikation (Modifikation 2) der Verbindung der Formel I 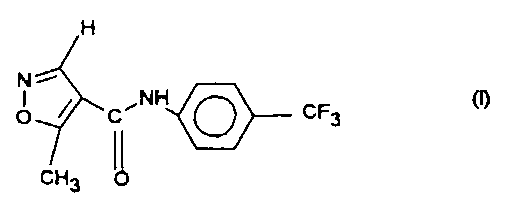 die im Röntgenbeugungsdiagramm in Transmission mit fokusierendem Debye-Scherrer-Strahlengang und Cu-K_{α1}-Strahlung Linien bei folgenden Beugungswinkeln 2Theta (°) aufweist
- Linien starker Intensität:: 10,65; 14,20; 14,80; 16,10; 21,70; 23,15; 24,40; 24, 85; 25,50; 25,85; 26,90; 29,85
- Linien mittlerer Intensität:: 7,40; 9,80; 13,10; 15,45; 16,80; 20,70; 21,45; 22,80; 23,85; 27,25; 28,95

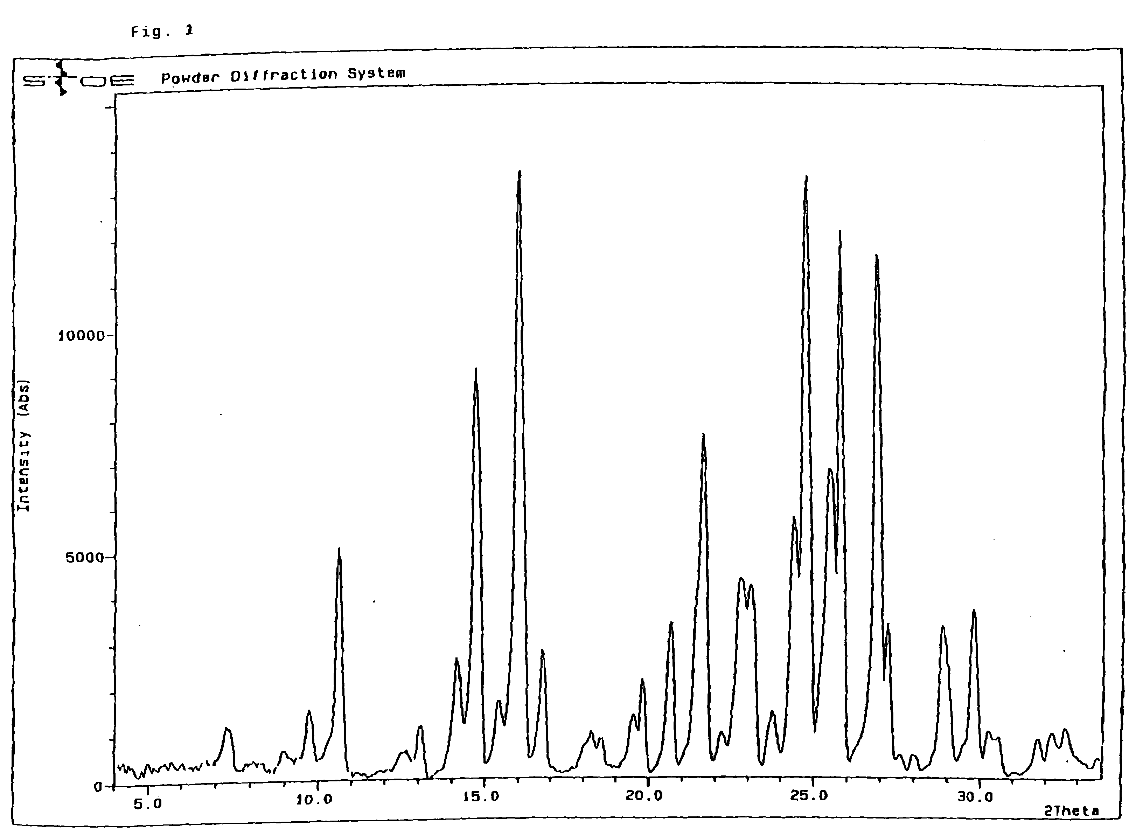

Das mit Cu-K_{α1}-Strahlung aufgenommene Röntgenbeugungsdiagramm der Modifikation 2 ist in Figur 1 dargestellt. Zur Aufnahme des Diagramms wurden das Zweikreisdiffraktometer STADI P der Firma Stoe (Darmstadt, Deutschland) oder das rechnergestützte Einkristalldiffraktometer R3 m/V der Firma Siemens (eingesetzte Strahlung Mo K_{α}) verwendet

Das Infrarot-Spektrum der Modifikation 2 der Verbindung der Formel I(1 mg in 300 mg KBr) aufgenommen mit einem Infrarot-Spektrophotometer zeigt die folgenden Hauptbanden (Einheit cm⁻¹):

| | | | |
|---|---|---|---|
| 1321 | 1481 | 672 | 3201 |
| 1607 | 3355 | 763 | 701 |
| 1109 | 1264 | 908 | 948 |
| 1065 | 1384 | 754 | 511 |
| 1536 | 1361 | 592 | 733 |
| 1663 | 852 | 427 | 960 |
| 1241 | 1014 | 3111 | 1779 |
| 1410 | 3297 | 3065 | 1811 |
| 1160 | 877 | 3221 | 484 |
| 1691 | 940 | 974 | 3442 |
| 831 | 3274 | 3129 | 3434 |
| 1188 | 894 | 628 | |

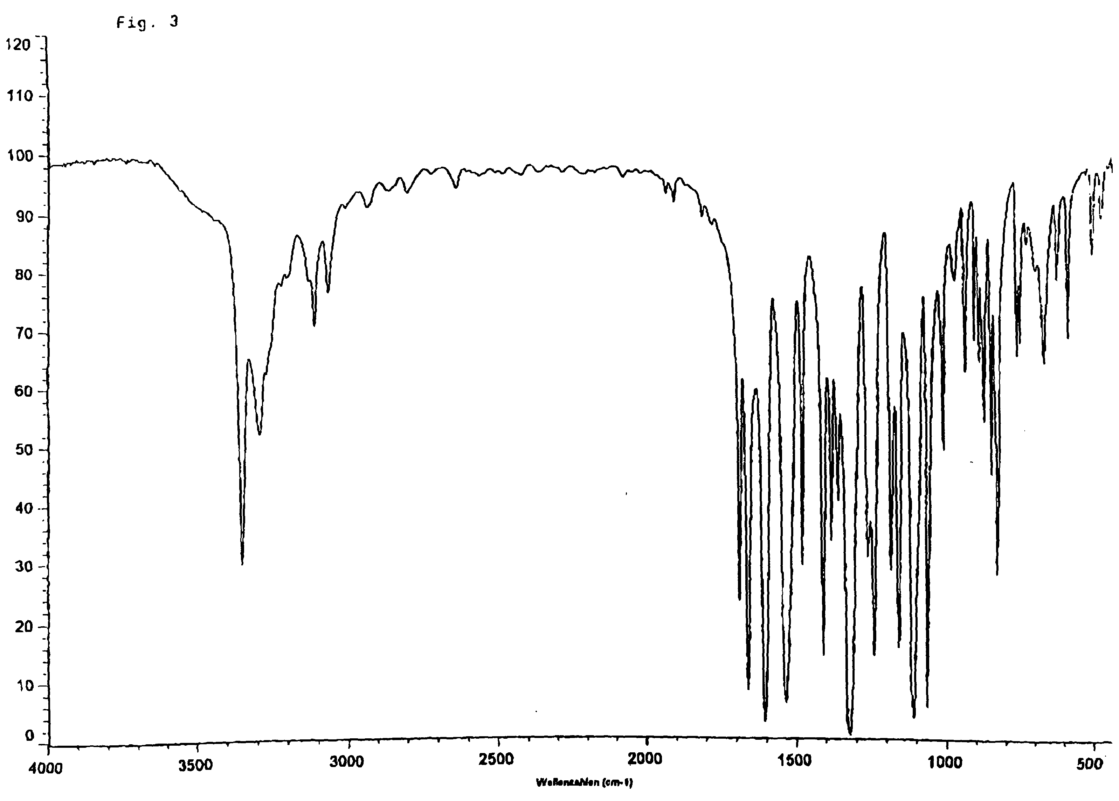

Die angegebenen Wellenzahlen sind in ansteigender Intensität angeordnet. Das Infrarot-Spektrum der Modifikation 2 der Verbindung der Formel I gemäß Beispiel 1 ist ferner in Figur 3 dargestellt, wobei in der Ordinate die Transmission in % und auf der Abszisse die Wellenzahl in cm ⁻¹ angegeben wurde.

Die Verbindung der Formel I kristallisiert in der Modifikation 2 in der Raumgruppe P2₁/c mit 8 Molekülen in der Elementarzelle. Die Moleküle der Verbindung der Formel I liegen als Dimere vor, die aus den Einzelmolekülen durch Bildung einer -C=O...HN-Wasserstoffbrückenbindung (2,938 Å) hervorgehen, wobei die zwei Molekülebenen praktisch senkrecht zueinander stehen (91,2°). Die beiden Moleküle haben eine stark unterschiedliche Konformation. Die Winkel der Fünf- und Sechsringe mit der zentralen Carbonylgruppe betragen 5,4 ° und 2,1 ° beziehungsweise 23,4 ° und 23,1°. Die letztere Verdrillung schafft die sterische Voraussetzungen dafür, daß die Wasserstoffbrückenbindung zwischen den beiden Molekülen möglich wird.

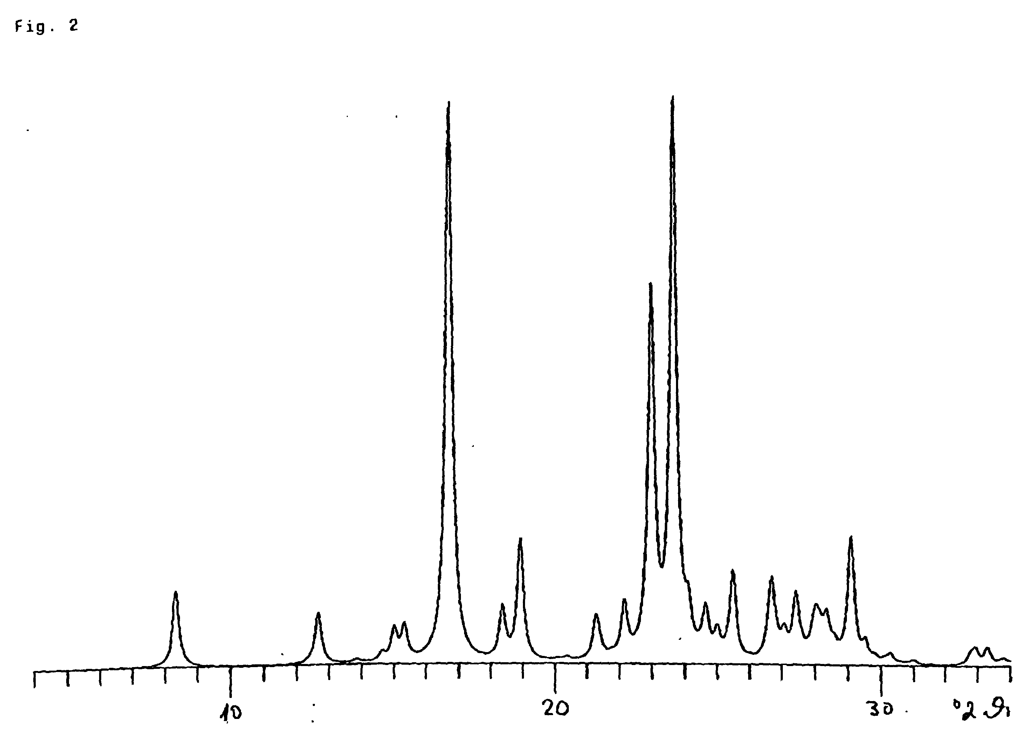

Die Verbindung der Formel I ist an sich bekannt und wird auch als Leflunomid (HWA 486) bezeichnet. Sie kann auf die in US 4,284,786 beschriebene Weise erhalten werden. Die durch Umkristallisation aus beispielsweise Toluol hergestellten Kristalle fallen jedoch in der Kristallmodifikation 1 an. Das Röntgenbeugungsdiagramm (Cu-K_{α1}Strahlung) der Modifikation 1 ist in Figur 2 wiedergegeben und weist charakteristische Linien bei folgenden Beugungswinkeln 2 Theta (°) auf:
- Linien starker Intensität:: 16,70; 18,90; 23,00; 23,65; 29,05
- Linien mittlerer Intensität:: 8,35; 12,65; 15,00; 15,30; 18,35; 21,25; 22,15; 24,10; 24,65; 25,45; 26,65; 27,40; 28,00; 28,30

Die Verbindung der Formel I kristallisiert in der Modifikation 1 in der Raumgruppe P2₁/c mit 4 Molekülen in der Elementarzelle. Das Molekül ist im wesentlichen planar. Der Winkel zwischen den planaren Atomgruppen ist kleiner als 2,4°. Die Moleküle sind im Kristall in Stapeln angeordnet Die Moleküle liegen in Stapeln nebeneinander und sind in einer antiparallelen Art angeordnet. Sehr schwache Wasserstoffbrückenbindungen verbinden die Dimere im Kristallverband (NH ... N: 3,1444 Å). Die C=O-Gruppe ist an keiner Wasserstoffbrückenbindung beteiligt.

Die Röntgenbeugungsdiagramme erlauben ferner die Bestimmung der Menge der Modifikation 2 in einer Mischung, enthaltend beide Modifikationen. Zur quantitativen Bestimmung eignet sich die Linie bei 8,35° 2 Theta der Modifikation 1 und die Linie bei 16,1° 2 Theta der Modifikation 2. Bildet man das Verhältnis der Peakhöhen und korreliert man es mit dem Gehalt an der Modifikation so erhält man eine Eichgerade. Die Nachweisgrenze dieser Methode liegt bei etwa 0,3 % Modifikation 2 in Kristallen mit Modifikation 1.

Die Modifikation 2 ist im Vergleich zur Modifikation 1 besser wasserlöslich. Bei 37 °C lassen sich 38 mg/l der Modifikation 2 lösen, während von Modifikation 1 25 mg/l in Lösung gehen. Femer ist die Modifikation 2 in dem Temperatürbereich von -15 °C bis +40 °C, bevorzugt von 20 °C bis 40 °C, stabil und wird nicht in Modifikation 1 umgewandelt

Die erfindungsgemäße Modifikation 2 der Verbindung der Formel I erhält man beispielsweise durch Erwärmen einer Suspension der Kristalle der Modifikation 1 oder Mischungen der Modifikationen 1 und 2 der Verbindung der Formel I in einem Lösungsmittel auf eine Temperatur von etwa 10 °C bis 40 °C, bevorzugt 15 °C bis 30 °C, insbesondere von 20 °C bis 25 °C. Die Herstellgeschwindigkeit ist im wesentlichen von der Temperatur abhängig. Vorteilhaft sind Lösemittel, in denen sich die Verbindung der Formel I schlecht löst. Beispielsweise kann Wasser verwendet werden oder wäßrige Lösungen mit (C₁-C₄)-Alkoholen, z.B. Methanol, Ethanol, Propanol, Isopropanol, Butanol oder Isobutanol und/oder mit Ketonen wie Aceton oder Methylethylketon . In der Regel erfolgt das Erwärmen in wäßriger Suspension, zweckmäßigerweise unter Rühren oder Schütteln. Die Temperaturbehandlung erfolgt so lange, bis die Modifikation 1 vollständig in die Modifikation 2 überführt ist

Die vollständige Überführung der Modifikation 1 in die Modifikation 2 ist von der Temperatur abhängig und dauert in der Regel bei einer Temperatur von 20 °C von 36 Stunden bis 65 Stunden, bevorzugt von 48 Stunden bis 60 Stunden. Die Kontrolle der Reaktion erfolgt röntgenographisch oder IR-spektroskopisch mittels während der Behandlung entnommener Proben.

Ein weiteres Verfahren zur Herstellung der Modifikation 2 der Verbindung der Formel I besteht darin, die Modifikation 1 oder Mischungen der Modifikationen 1 und 2 in einem Lösungsmittel zu lösen und dann schockartig auf Temperaturen von etwa -5 °C bis -25 °C abzukühlen. Als Lösungsmittel geeignet sind beispielsweise mit Wasser mischbare Lösungsmittel wie (C₁-C₄)-Alkohole, aber auch Ketone wie Aceton oder Methylethylketon oder andere Lösungsmittel wie Ethylacetat, Toluol oder Dichlormethan. Der Lösungsvorgang erfolgt bei Raumtemperatur von 20 °C bis 25 °C oder auch erhöhten Temperaturen bis zum Siedepunkt des Lösemittels unter Normaldruck oder unter erhöhten oder verminderten Druck. Die erhaltene Lösung wird gegebenenfalls filtriert um ungelöste Bestandteile oder Kristalle von Leflunomid abzutrennen. Danach wird die filtrierte Lösung so schnell abgekühlt, daß sich nur Kristalle der Modifikation 2 bilden. Ein ausreichendes Abkühlverfahren besteht beispielsweise darin, daß die filtrierte Lösung in ein Gefäß, das auf -15 °C gekühlt ist, eingebracht wird, oder durch Versprühen der filtrierten Lösung in einen auf -10 °C gekühlten Raum, oder Abkühlung der Lösung unter Vakuumkondensationsbedingungen. Ein bevorzugtes Verfahren besteht darin die Verbindung der Formel I in Methanol einzubringen und den Lösungsvorgang bei Siedetemperatur von Methanol unter Normaldruck oder verminderten Druck durchzuführen; anschließend die heiße Lösung zu filtrieren und die filtrierte Lösung so langsam in ein Gefäß, das auf -15 °C gekühlt ist, zu überführen, daß die Temperatur der erhaltenen Kristallsuspension auf nicht mehr als -10 °C steigt Die ausgefallenen Kristalle werden dann mehrfach mit Methanol gewaschen und unter verminderten Druck getrocknet Die Kristallisation kann ohne Animpfen mit Kristallen der Modifikation 2 erfolgen oder bevorzugt durch Animpfen mit Kristallen der Modifikation 2. Das Animpfen erfolgt im abgekühlten Gefäß. Die Menge des Animpfmaterials hängt von der Menge der Lösung ab und kann von einem Fachmann leicht ermittelt werden. Die obengenannten Verfahren eignen sich auch zur Umwandlung von Mischungen, enthaltend Modifikation 1 und 2, in eine im wesentlichen reine Modifikation 2 der Verbindung der Formel I.

Die erfindungsgemäße Modifikation 2 der Verbindung der Formel I eignet sich beispielsweise zur Behandlung von
- akuten immunologischen Ereignissen wie Sepsis, Allergie, Graft-versus-Host- und Host-versus-Graft-Reaktionen
- Autoimmunerkrankungen, insbesondere rheumatoide Arthritis, systemischem Lupus erythematodes, multipler Sklerose
- Psoriasis, atopischer Dermatitis, Asthma, Urtikaria, Rhinitis, Uveitis
- Typ II-Diabetes
- Leberfibrose, zystischer Fibrose, Kolitis
- Krebserkrankungen wie Lungenkrebs, Leukämie, Eierstockkrebs, Sarkome, Kaposi's Sarkom, Meningiom, Darmkrebs, Lymphknotenkrebs, Himtumore, Brustkrebs, Pankreaskrebs, Prostatakrebs oder Hautkrebs.

Die Erfindung betrifft auch Arzneimittel, gekennzeichnet durch einen wirksamen Gehalt an der Modifikation 2 der Verbindung der Formel I, zusammen mit einem pharmazeutisch geeigneten und physiologisch verträglichen Trägerstoff, Zusatzstoff und/oder Wirk- und Hilfsstoffen.

Die erfindungsgemäßen Arzneimittel, gekennzeichnet durch einen wirksamen Gehalt an der Modifikation 2 der Verbindung der Formel I, zeigen die gleiche Wirksamkeit an Menschen, die an rheumatischer Arthritis leiden, im Vergleich mit der Behandlung mit einem Arzneimittel, gekennzeichnet durch einen wirksamen Gehalt an der Modifikation 1 der Verbindung der Formel I.

Die Erfindung betrifft ferner ein Verfahren zur Herstellung des Arzneimittels, das dadurch gekennzeichnet ist, daß man die Modifikation 2 der Verbindung der Formel I und einen pharmazeutischen Träger zu einer pharmazeutischen Darreichungsform verarbeitet.

Das erfindungsgemäße Arzneimittel kann als Dosiereinheit in Form von Arzneiformen wie Kapseln (einschließlich Mikrokapseln), Tabletten (einschließlich Dragees und Pillen) oder Zäpfchen vorliegen, wobei bei Verwendung von Kapseln das Kapselmaterial die Funktion des Trägers wahrnehmen und der Inhalt z.B. als Pulver, Gel, Emulsion, Dispersion oder Suspension vorliegen kann. Besonders vorteilhaft und einfach ist es jedoch, orale (perorale) Formulierungen mit der Modifikation 2 der Verbindung der Formel I herzustellen, die die berechneten Menge des Wirkstoffs zusammen mit einem pharmazeutischen Träger enthalten. Auch eine entsprechende Formulierung (Zäpfchen) für die rektale Therapie kann angewandt werden. Ebenso ist die transdermale Applikation in Form von Salben, Cremes oder orale Applikation von Tabletten oder Suspensionen, die die erfindungsgemäße Zubereitung enthalten, möglich.

Salben, Pasten, Cremes und Puder können neben den Wirkstoffen die üblichen Trägerstoffe enthalten, z.B. tierische und pflanzliche Fette, Wachse, Paraffine, Stärke, Traganth, Zellulosederivate, Polyethylenglykole, Silicone, Bentonite, Talkum, Zinkoxid, Milchzucker, Kieselsäure, Aluminiumhydroxid, Kalziumsilikat und Polyamidpulver oder Gemische dieser Stoffe.

Die Tabletten, Pillen oder Granulatkörper können nach üblichen Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z.B. Kartoffel-, Mais- oder Weizenstärke), Cellulose wie Ethylcellulose, Siliziumdioxid, verschiedene Zucker wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Celluloseester, oberflächenaktive Substanzen, Weichmacher, Pigmente und ähnliche Zusätze entsprechend dem Stand der Technik. Zur Herstellung der Zubereitungen kann jedes übliche Fließregulierungs-, Schmier- oder Gleitmittel wie Magnesiumstearat und Trennmittel verwendet werden.

Die anzuwendende Dosierung ist selbstverständlich abhängig von verschiedenen Faktoren wie dem zu behandelnden Lebewesen (d.h. Mensch oder Tier), Alter, Gewicht, allgemeiner Gesundheitszustand, dem Schweregrad der Symptome, der zu behandelnden Erkrankung, der Art der begleitenden Behandlung mit anderen Arzneimitteln, oder der Häufigkeit der Behandlung. Die Dosierungen werden im allgemeinen mehrfach pro Tag und vorzugsweise einmal bis dreimal pro Tag verabreicht.

Die geeignete Therapie besteht somit z.B. in der Verabreichung von einer, zwei oder 3 Einzeldosierungen einer Zubereitung bestehend aus N-(4-Trifluormethylphenyl)-5-methyl-isoxazol-4-carboxamid in der Modifikation 2 in einer Menge von 2 bis 150 mg, bevorzugt 10 bis 100 mg, insbesondere 10 bis 50 mg.

Die Menge der Wirkkomponenten hängt naturgemäß von der Zahl der Einzeldosierungen und auch von der zu behandelnden Krankheit ab. Die Einzeldosierung kann auch aus mehreren, gleichzeitig verabreichten Dosiereinheiten bestehen.

### Beispiel 1

### Herstellung der Modifikation 2

Etwa 40 mg der Verbindung der Formel I hergestellt gemäß US 4,284,786 wurden mit 40 ml Wasser in Flaschen (Volumen 45 ml) geschüttelt. Das Schütteln der verschlossenen Flaschen erfolgte bei 15 - 25 °C im Wasserbad. Nach 48 Stunden wurde eine Probe entnommen, gefiltert, getrocknet und ein Pulver-Röntgen-Beugungsdiagramm angefertigt. Die Messung erfolgte mit dem Zweikreisdiffraktometer STADI P der Firma Stoe (Darmstadt, Deutschland) mit Cu-K_{α1}-Strahlung im Debye-Scherrer Strahlengang und Transmission.

Fig. 1 zeigt das erhaltene Röntgenbeugungsdiagramm und ist typische für die Modifikation 2 der Verbindung der Formel I.

### Beispiel 2

### Löslichkeit in Wasser

| | |
|---|---|
| Apparat | Standkolben, Magnetrührer, Wasserbad 37 °C ± 0,5 °C |
| Medium | Wasser (+37 °C) |
| Probennahme | 5 Stunden |
| Präparation | Die Modifikation 1 und 2 gemäß der Beispiele 1 und 2 wurde in Wasser überführt und bei 37 °C stark gerührt |
| Detektion | UV-Spektroskopie bei der Wellenlänge 258 µm |

| Ergebnis: | |
|---|---|
| Modifikation 1 | 25 mg lösen sich in 1 Liter Wasser bei 37 °C |
| Modifikation 2 | 38 mg lösen sich in 1 Liter Wasser bei 37 °C |

### Beispiel 3

### Stabilität der Modifikation

Proben der Modifikation 2 wurden wie in Beispiel 1 hergestellt und bei verschiedenen Temperaturen und Luftfeuchtigkeit gelagert Nach den genannten Zeiten wurden Proben entnommen und ein Röntgenbeugungsdiagramm wurde wie in Beispiel 1 angefertigt. Tabelle 1 zeigt die Ergebnisse.

**Tabelle 1:**

| Zeit (Monate) | Lagerbedingungen | Kristallmodifikation |
|---|---|---|
| 1 | -15°C | 2 |
| 3 | -15°C | 2 |
| 6 | -15°C | 2 |
| 1 | +25°C | 2 |
| 3 | +25°C | 2 |
| 6 | +25°C | 2 |
| 1 | +40°C | 2 |
| 3 | +40°C | 2 |
| 6 | +40°C | 2 |
| 1 | +40°C/75 % relative Feuchtigkeit | 2 |
| 3 | +40°C/75% relative Feuchtigkeit | 2 |
| 6 | +40°C/75% relative Feuchtigkeit | 2 |
| 1 | +60°C | etwa 76 % 1¹⁾ |
| 3 | +60°C | 1 |

| | | |
|---|---|---|
| ¹⁾ Für die Bestimmung der Modifikation 1 wurde eine Eichkurve verwendet. Zur Erstellung der Eichkurve für die quantitative Bestimmung wurde für die Phase 1 der Reflex bei 8,35°2ϑ und für die Phase 2 der Reflex bei 16,1°2ϑ verwendet. Es wurden die Verhältnisse der entsprechenden Peakhöhen gebildet und mit den Gehalten an Phase 2 korreliert. Die Grenze der Methode liegt bei 0,3 %. Die Probe bei +60°C und Lagerung für 1 Monat enthält nach dieser Methode etwa 76 % der Modifikation 1. | | |

### Beispiel 4

### Herstellung der Modifikation 1

Wasserfeuchtes Leflunomid, wird roh zunächst in Isopropanol/Wasser gelöst (entsprechend 16 kg Leflunomid, roh, trocken, in 28 l Isopropanol plus die Menge Wasser, die zusammen mit dem Wassergehalt des feuchten Produktes eine Gesamt-Wassermenge von 9 l ergibt).

Anschließend wird auf 78°C bis 82°C erwärmt, 25 Minuten (min) bei dieser Temperatur gerührt und dann über einen Drucktrichter in einen ebenfalls schon auf gleiche Temperatur geheizten Kessel filtriert. Der Drucktrichter wird mit der Menge Isopropanol nachgespült, die zusammen mit eingesetztem Isopropanol (iPrOH) ein Verhältnis von iPrOH/Wasser von 4:1 ergeben (hier 4 l). Danach wird ebenfalls auf 78°C bis 82°C vorgeheiztes Wasser zugesetzt (32 l, ergibt iPrOH/Wasser = 0,8:1). Dabei trübt sich die Lösung bereits, die dann in 20 min auf etwa 65°C gekühlt, etwa 40 min bei dieser Temperatur gehalten, danach in 70 min auf etwa 40°C gekühlt und noch 20 min nachgerührt wird. Das Produkt wird durch Zentrifugation isoliert.

Tabelle 1 zeigt die Ergebnisse von 4 Ansätzen.

**Tabelle 1:**

| Ansatz | Ausgangs-Konzentration | Verhältnis iPrOH/H₂O | Endkonzentration | Anteil* Kristalle Modifikation 2 | Ausbeute |
|---|---|---|---|---|---|
| | [g/l] | | [g/l] | [%] | [%] |
| 1 | 600 | 4:1 | 600 | n.b. | 73,2 |
| 2 | 600 | 3:1 | 563 | <0,4 | 71,4 |
| 3 | 400 | 2:1 | 333 | <0,4 | 70,5 |
| 4 | 400 | 0,8:1 | 222 | <0,4 | 85,6 |

| | | | | | |
|---|---|---|---|---|---|
| * Die Bestimmung erfolgte durch Röntgen-Pulverdiffraktometrie; der Anteil der Modifikation 2 war immer unterhalb der Nachweisgrenze, die bei etwa 0,4 % liegt. n.b. bedeutet nicht bestimmt | | | | | |

## Patentansprüche

1. Modifikation 2 der Verbindung der Formel I 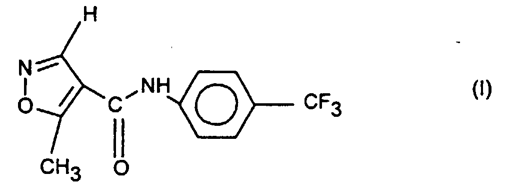 die im Röntgenbeugungsdiagramm in Transmission mit fokusierendem Debye-Scherrer-Strahlengang und Cu-K_{α1}-Strahlung Linien bei folgenden Beugungswinkeln 2Theta (°) aufweist:
Linien starker Intensität: 10,65; 14,20; 14,80; 16,10; 21,70; 23,15; 24,40; 24,85; 25,50; 25,85; 26,90; 29,85
Linien mittlerer Intensität: 7,40; 9,80; 13,10; 15,45; 16,80; 20,70; 21,45; 22,80; 23,85; 27,25; 28,95.

2. Verfahren zur Herstellung der Modifikation 2 der Verbindung der Formel I gemäß Anspruch 1, dadurch gekennzeichnet, daß eine Lösung, enthaltend die Verbindung der Formel I, die nicht in der Modifikation 2 vorliegt oder Mischungen der Modifikation 1 und 2, schockartig auf eine Temperatur von weniger als -5°C bis -25°C abgekühlt wird.

3. Verfahren zur Herstellung der Modifikation 2 der Verbindung der Formel I gemäß Anspruch 1, dadurch gekennzeichnet, daß eine Suspension, enthaltend die Verbindung der Formel I, die nicht in der Modifikation 2 vorliegt oder Mischungen der Modifikation 1 und 2, auf eine Temperatur von 10 °C bis 40 °C erwärmt wird.

4. Verfahren gemäß Anspruch 3, dadurch gekennzeichnet, daß eine wäßrige Suspension erwärmt wird.

5. Verfahren gemäß Anspruch 2, dadurch gekennzeichnet, daß als Lösemittel mit Wasser mischbare Lösungsmittel wie (C₁-C₄)-Alkohole, zB. Methanol, Ethanol, Propanol, Isopropanol, Butanol oder Isobutanol, insbesondere Isopropanol, aber auch Ketone wie Aceton oder Methylethylketon, oder Mischungen der Lösemittel mit Wasser oder nicht wassermischbare Lösungsmittel wie Ethylacetat, Toluol oder Dichlormethan eingesetzt werden.

6. Verfahren gemäß der Ansprüche 2 und 5, dadurch gekennzeichnet, daß eine wäßrige Mischung mit etwa 40 % bis 90 % Isopropanol eingesetzt wird.

7. Verfahren gemäß der Ansprüche 2, 5 und 6, dadurch gekennzeichnet, daß die Kristallisation in Anwesenheit von Kristallen der Modifikation 2 der Verbindung der Formel I durchgeführt wird.

8. Arzneimittel , gekennzeichnet durch einen Gehalt an der Modifikation 2 der Verbindung der Formel I gemäß Anspruch 1 und einem physiologisch akzeptablen Träger.

9. Verwendung der Modifikation 2 der Verbindung der Formel I gemäß Anspruch 1 zur Herstellung eines Arzneimittels zur Behandlung von akuten immunologischen Ereignissen wie Sepsis, Allergie, Graft-versus-Host- und Host-versus-Graft-Reaktionen, Autoimmunerkrankungen, insbesondere rheumatoide Arthritis, systemischem Lupus erythematodes, multipler Sklerose, Psoriasis, atopischer Dermatitis, Asthma, Urtikaria, Rhinitis, Uveitis, Typ II-Diabetes, Leberfibrose, zystischer Fibrose, Kolitis, Krebserkrankungen wie Lungenkrebs, Leukämie, Eierstockkrebs, Sarkome, Kaposi's Sarkom, Meningiom, Darmkrebs, Lymphknotenkrebs, Hirntumore, Brustkrebs, Pankreaskrebs, Prostatakrebs oder Hautkrebs.

## Claims

1. A Modification 2 of the compound of the formula I 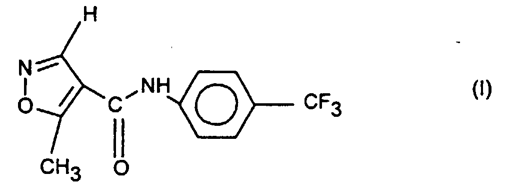 which, in the transmission X-ray diffraction pattern obtained with a focusing Debye-Scherrer beam and Cu-K_{α1}-radiation, has lines at the following diffraction angles 2 theta (°):
Lines of strong intensity: 10.65; 14.20; 14.80; 16.10; 21.70; 23.15; 24.40; 24.85; 25.50; 25.85; 26.90; 29.85
Lines of medium intensity: 7.40; 9.80; 13.10; 15.45; 16.80; 20.70; 21.45; 22.80; 23.85; 27.25; 28.95.

2. A process for the preparation of Modification 2 of the compound of the formula I as claimed in claim 1, wherein a solution containing the compound of the formula I which is not present in Modification 2 or mixtures of Modifications 1 and 2 is cooled abruptly to a temperature of less than -5°C to -25°C.

3. The process for the preparation of Modification 2 of the compound of the formula I as claimed in claim 1, wherein a suspension containing the compound of the formula I which is not present in Modification 2 or mixtures of Modifications 1 and 2 is heated to a temperature of from 10°C to 40°C.

4. The process as claimed in claim 3, wherein an aqueous suspension is heated.

5. The process as claimed in claim 2, wherein the solvents used are water-miscible solvents, such as (C₁-C₄)alcohols, e.g. methanol, ethanol, propanol, isopropanol, butanol or isobutanol, in particular isopropanol, as well as ketones, such as acetone or methyl ethyl ketone, or mixtures of the solvents with water or water-immiscible solvents, such as ethyl acetate, toluene or dichloromethane.

6. The process as claimed in claims 2 and 5, wherein an aqueous mixture containing from about 40 % to 90 % of isopropanol is used.

7. The process as claimed in claims 2, 5 and 6, wherein the crystallization is carried out in the presence of crystals of Modification 2 of the compound of the formula I.

8. A drug containing Modification 2 of the compound of the formula I as claimed in claim 1 and a physiologically acceptable excipient.

9. The use of Modification 2 of the compound of the formula I as claimed in claim 1 for the preparation of a drug for the treatment of acute immunological episodes, such as sepsis, allergies, graft-versus-host- and host-versus-graft-reactions, autoimmune diseases, in particular rheumatoid arthritis, systemic lupus erythematosus, multiple sclerosis, psoriasis, atopic dermatitis, asthma, urticaria, rhinitis, uveitis, type II diabetes, liver fibrosis, cystic fibrosis, colitis, cancers, such as lung cancer, leukemia, ovarian cancer, sarcomas, Kaposi's sarcoma, meningioma, intestinal cancer, lymphatic cancer, brain tumors, breast cancer, pancreatic cancer, prostate cancer or skin cancer.

## Revendications

1. Forme 2 du composé de formule I 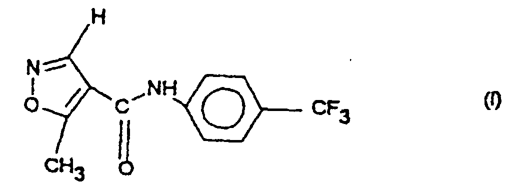 qui, dans le diagramme de diffraction des rayons X en transmission sous une trajectoire d'un faisceau avec la méthode de Debye-Scherrer focalisante et avec la radiation K_{α1} du Cu, présente des raies aux angles de diffraction 2θ (°) suivants:
raies de forte intensité : 10,65; 14,20; 14,80; 16,10; 21,70; 23,15; 24,40; 24,85; 25,50; 25,85; 26,90; 29,85
raies d'intensité moyenne: 7,40; 9,80; 13,10; 15,45; 16,80; 20,70; 21,45; 22,80; 23,85; 27,25; 28,95.

2. Procédé pour la préparation de la forme 2 du composé de formule I selon la revendication 1, caractérisé en ce que l'on refroidit brusquement jusqu'à une température de moins de -5°C à -25°C une solution contenant le composé de formule I qui ne se trouve pas sous la forme 2, ou des mélanges des formes 1 et 2.

3. Procédé pour la préparation de la forme 2 du composé de formule I selon la revendication 1, caractérisé en ce que l'on chauffe à une température de 10 à 40°C une suspension contenant le composé de formule I qui ne se trouve pas sous la forme 2, ou des mélanges des formes 1 et 2.

4. Procédé selon la revendication 3, caractérisé en ce que l'on chauffe une suspension aqueuse.

5. Procédé selon la revendication 2, caractérisé en ce que l'on utilise comme solvant des solvants miscibles à l'eau, tels que des alcools en C₁-C₄, par exemple le méthanol, l'éthanol, le propanol, l'isopropanol, le butanol ou l'isobutanol, en particulier l'isopropanol, mais également des cétones telles que la cétone ou la méthyléthylcétone, ou des mélanges des solvants avec l'eau, ou des solvants non miscibles à l'eau, tels que l'acétate d'éthyle, le toluène ou le dichlorométhane.

6. Procédé selon les revendications 2 et 5, caractérisé en ce que l'on utilise un mélange aqueux contenant environ 40 % à 90 % d'isopropanol.

7. Procédé selon les revendications 2, 5 et 6, caractérisé en ce que l'on effectue la cristallisation en présence de cristaux de la forme 2 du composé de formule I.

8. Médicament, caractérisé par une teneur en la forme 2 du composé de formule I selon la revendication 1, et en un véhicule physiologiquement acceptable.

9. Utilisation de la forme 2 du composé de formule I selon la revendication 1, pour la fabrication d'un médicament destiné au traitement d'incidents immunologiques aigus tels que la septicémie, l'allergie, des réactions du greffon contre l'hôte et de l'hôte contre le greffon, de maladies auto-immunes, en particulier la polyarthrite rhumatoïde, le lupus érythémateux disséminé, la sclérose en plaques, le psoriasis, la dermatite atopique, l'asthme, l'urticaire, la rhinite, l'uvéite, le diabète de type II, la fibrose hépatique, la fibrose kystique, la colite, de maladies cancéreuses telles que le cancer du poumon, la leucémie, le cancer de l'ovaire, des sarcomes, le sarcome de Kaposi, le méningiome, le cancer de l'intestin, le cancer des ganglions lymphatiques, les tumeurs du cerveau, le cancer du sein, le cancer du pancréas, le cancer de la prostate ou le cancer de la peau.
